# EUROPEAN PATENT APPLICATION

(11) **EP 2 343 054 A1**
(43) Date of publication of application: **13.07.2011**
(21) Application number: 10150029.6
(22) Date of filing: 04.01.2010
(51) Int. Cl.: A61K 9/16, A61K 9/50, A61K 31/22

(54) **Pellets and microparticles of pravastatin sodium and a process of making them**

(71) Applicant: LEK Pharmaceuticals d.d., 1526 Ljubljana (SI)
(72) Inventor: The designation of the inventor has not yet been filed
(74) Representative: Hodzar, Damjan

(57) **Abstract**

The present invention belongs to the field of pharmaceutical industry and relates to a pharmaceutical composition comprising one or more cores, an enteric coating and a non-acidic protective coating between the core and the enteric coating comprising polymorphic pravastatin sodium. Furthermore, the present invention relates to pharmaceutical compositions comprising one or more cores comprising pravastatin sodium and specific excipients, and to a process for the preparation of said cores. Moreover, the present invention relates to a dosage form comprising said pharmaceutical compositions and to the use of said dosage form for the prevention or treatment of hypercholesterolemia and hyperlipidemia.

## Description

### Field of the invention

The present invention belongs to the field of pharmaceutical industry and relates to a pharmaceutical composition comprising one or more cores, an enteric coating and a non-acidic protective coating between the core and the enteric coating comprising pravastatin sodium of specific polymorphic structure. Furthermore, the present invention relates to pharmaceutical compositions comprising one or more cores comprising pravastatin sodium and specific excipients, and to a process for the preparation of said cores. Moreover, the present invention relates to a dosage form comprising said pharmaceutical compositions and to the use of said dosage form for the prevention or treatment of hypercholesterolemia and hyperlipidemia.

### Description of the background art

Pravastatin, chemically known as (+)- (3R, 5R) -3,5-dihydroxy-7- [(1 S, 2S, 6S, 8S, 8aR)-6-hydroxy-2-methyl-8-[(S)-2-methylbutyryloxy]-1, 2,6, 7,8, 8a- hexahydro-1-naphthyl] heptanoate, is an 3-hydroxy-3-methylglutaryl-coenzyme A (HMG-CoA) reductase inhibitor which reduces plasma cholesterol levels by inhibiting de novo cholesterol synthesis and increasing the receptor mediated catabolism of low density lipoproteins. The drug exhibits hepatocellular tissue selectivity, with greatest inhibition of cholesterol synthesis occurring in the liver and thereby inhibiting the unwarranted effects on cholesterol synthesis in non-hepatic (peripheral) cells. Its favorable effects on cardiovascular morbidity and total mortality renders it as an effective alternative to currently used HMG-CoA reductase inhibitors for patients with elevated cholesterol levels, multiple risk factors or coronary heart disease. Pravastatin and its respective salts, respectively, exists in many polymorphic forms.

Pravastatin sodium is a relatively polar hydrophilic compound with a partition coefficient (octanol/water) of 0.23 at a pH of 7.0. US 6,740,775, US 7,262,218 and WO 01/43723 disclose different polymorphic forms of Pravastatin sodium. US 6,740,775 discloses pravastatin sodium "form LEK" showing characteristic peaks in a X-ray diffractogram at 2 θof 4.0, 6.3, 10.2, 11.8, 16.3, 17.3, 18.0 and 20.0 +/- 0.2° of the 2 θ values.

When administered, pravastatin is rapidly absorbed, with peak plasma levels of parent compound attained after 1 to 1.5 hours following ingestion. The average oral absorption of pravastatin is 34% and absolute bioavailability is 17%. Pravastatin undergoes extensive first pass extraction in the liver, which is its primary site of action, and the primary site of cholesterol synthesis and of LDL-C clearance. Pravastatin, like other HMG-CoA reductase inhibitors, has a variable bioavailability. The coefficient of variation (CV), based on between-subject variability, can be as high as 50% to 60%.

Low bioavailability of pravastatin is caused also due to extensive degradation in acidic environment in stomach. The drug shows poor stability in acidic conditions. If kept unprotected, pravastatin undergoes non-enzymatic conversion in the stomach to a relatively inactive metabolite. Consequently, a significant portion of the active ingredient never reaches the site of action in the liver.

As to pharmaceutical compositions comprising pravastatin, WO 2003080026 A1 discloses an oral drug delivery system for the controlled release of pravastatin comprising a core comprising pravastatin or its pharmaceutically acceptable salts and a water swellable polymer; an inert subcoating surrounding the core comprising at least one film forming polymer; and a coating of an enteric polymer over said subcoating, such that the system provides enhanced stability in the acidic environment of the stomach and exhibits controlled release of the drug. The pharmaceutical composition can be prepared for instance as tablets or beads, following the given general description of this technological procedure.

WO 00/33821 discloses an enteric coated pravastatin bead formulation which optionally includes a basifying agent. The pravastatin beads were prepared by using a mixture of pravastatin and microcrystalline cellulose with water.

Despite the above described pharmaceutical compositions containing pravastatin, there is still a need and thus an object for improved pharmaceutical compositions containing pravastatin and dosage forms comprising pravastatin, for its preparation and use.

### Summary of the invention

The present invention provides the following aspects, subject-matters and preferred embodiments, which respectively taken alone or in combination, contribute to solving the object of the present invention:
(1) A pharmaceutical composition comprising one or more cores, an acidic enteric coating and a non-acidic protective coating between the core(s) and the enteric coating, wherein the core(s) has (have) an average size of equal to or less than about 3000 µm, preferably an average size in the range of from about 3000 µm to about 50 µm, more preferably of from about 2000 µm to about 70 µm, and even more preferably from about 1500 µm to about 100 µm, and wherein the core(s) comprise(s) pravastatin sodium in a form showing characteristic peaks in an X-ray diffractogram at 2 θ of 4.0, 6.3, 10.2, 11.8, 16.3, 17.3, 18.0 and 20.0 +/- 0.2°, preferably +/- 0.1 ° of the 2 θ values ("form LEK").

In a further preferred embodiment, the cores have an average size in the range of from about 400 µm to about 50 µm, preferably in the range of from about 350 µm to about 75 µm, more preferably in the range of from about 350 µm to about 100 µm, more preferably in the range of from about 350 µm to about 120 µm, more preferably in the range of from about 320 µm to about 150 µm, and most preferably in the range of from 300 µm to about 150 µm.

Within the meaning of the present invention, the term "characteristic peaks" denotes the peaks exhibiting the highest intensity at the number of measurements.
References to 2 θ values are to those measured using CuKα radiation.
(2) The pharmaceutical composition according to item (1), wherein the core is coated with the non-acidic protective coating, and wherein the core with said non-acidic protective coating is coated with the acidic enteric coating.
(3) The pharmaceutical composition according to items (1) or (2), wherein said core is in the form of a pellet or of a microparticle.
(4) The pharmaceutical composition according to any of the preceding items, wherein if said core is in the form of a pellet the pellet has an average size in the range of from about 3000 µm to about 400 µm, preferably in the range of from about 2500 µm to about 400 µm, more preferably in the range of from about 2000 µm to about 400 µm, more preferably in the range of from about 1800 µm to about 500 µm, more preferably in the range of from about 1600 µm to about 600 µm, more preferably in the range of from about 1500 µm to about 650 µm, more preferably in the range of from about 1400 µm to about 700 µm, and most preferably in the range of from 1300 µm to about 700 µm,
   or wherein if said core is in the form of a microparticle the microparticle has an average size in the range of from less than 400 µm to about 50 µm, preferably in the range of from about 350 µm to about 75 µm, more preferably in the range of from about 350 µm to about 100 µm, more preferably in the range of from about 350 µm to about 120 µm, more preferably in the range of from about 320 µm to about 150 µm, and most preferably in the range of from 300 µm to about 150 µm.
(5) The pharmaceutical composition according to any of items (3) or (4), wherein the core comprises pravastatin sodium, and, additionally, hydroxypropylcellulose or crospovidone; in a further embodiment, the core preferably comprises pravastatin sodium, hydroxypropylcellulose, magnesium oxide, and magnesium stearate.

In a preferred embodiment of item (5), the core is in the form of a microparticle.
(6) The pharmaceutical composition according to item (5), wherein the core comprises pravastatin sodium in an amount of 20-60 wt.-%, hydroxypropylcellulose in an amount of 20-60 wt.-%, magnesium oxide in an amount of 5-30 wt.-%, and magnesium stearate in an amount of 5-30 wt.-%, based on the total weight of the core.
(7) The pharmaceutical composition according to any of items (3) or (4), wherein the core comprises pravastatin sodium, and, additionally, hydroxypropylcellulose or crospovidone; in a further embodiment, the core preferably comprises pravastatin sodium, crospovidone, lactose (such as lactose monohydrate; within the meaning of the present invention, the term "lactose" refers to anhydrous lactose and lactose monohydrate), magnesium oxide, and povidone.

In a preferred embodiment of item (7), the core is in the form of a pellet.
(8) The pharmaceutical composition according to item (7), wherein the core comprises pravastatin sodium in an amount of 10-40 wt.-%, crospovidone in an amount of 15-50 wt.-%, lactose in an amount of 20-60 wt.-%, magnesium oxide in an amount of 1-20 wt.-%, and povidone in an amount of 5-20 wt.-%, based on the total weight of the core.
(9) A pharmaceutical composition comprising one or more cores, an acidic enteric coating and a non-acidic protective coating between the core and the enteric coating, wherein the core comprises crystalline pravastatin sodium, crospovidone, lactose (such as lactose monohydrate; within the meaning of the present invention, the term "lactose" refers to anhydrous lactose and lactose monohydrate), magnesium oxide, and povidone,
   wherein the core is coated with the non-acidic protective coating, and
   wherein the core with said non-acidic protective coating is coated with the enteric coating.
(10) The pharmaceutical composition according to item (9), wherein the core comprises pravastatin sodium in an amount of 10-40 wt.-%, crospovidone in an amount of 15-50 wt.-%, lactose in an amount of 20-60 wt.-%, magnesium oxide in an amount of 1-20 wt.-%, and povidone in an amount of 5-20 wt.-%, based on the total weight of the core.
(11) The pharmaceutical composition according to items (9) or (10),
   wherein the core has an average size in the range of from about 3000 µm to about 400 µm, preferably in the range of from about 2500 µm to about 400 µm, more preferably in the range of from about 2000 µm to about 400 µm, more preferably in the range of from about 1800 µm to about 500 µm, more preferably in the range of from about 1600 µm to about 600 µm, more preferably in the range of from about 1500 µm to about 650 µm, more preferably in the range of from about 1400 µm to about 700 µm, and most preferably in the range of from 1300 µm to about 700 µm.
(12) The pharmaceutical composition according to any of items (9) to (11), wherein said core is in the form of a pellet.
(13) A pharmaceutical composition comprising one or more cores, an acidic enteric coating and a non-acidic protective coating between the core and the enteric coating, wherein the core comprises crystalline pravastatin sodium, hydroxypropylcellulose, magnesium oxide, and magnesium stearate,
   wherein the core is coated with the non-acidic protective coating, and
   wherein the core with said non-acidic protective coating is coated with the enteric coating.
(14) The pharmaceutical composition according to item (13), wherein the core comprises pravastatin sodium in an amount of 20-60 wt.-%, hydroxypropylcellulose in an amount of 20-60 wt.-%, magnesium oxide in an amount of 5-30 wt.-%, and magnesium stearate in an amount of 5-30 wt.-%, based on the total weight of the core.
(15) The pharmaceutical composition according to items (13) or (14),
   wherein the core has an average size in the range of from about 400 µm to about 50 µm, preferably in the range of from about 350 µm to about 75 µm, more preferably in the range of from about 350 µm to about 100 µm, more preferably in the range of from about 350 µm to about 120 µm, more preferably in the range of from about 320 µm to about 150 µm, and most preferably in the range of from 300 µm to about 150 µm.
(16) The pharmaceutical composition according to any of items (13) to (15), wherein said core is in the form of a microparticle.
(17) The pharmaceutical composition according to any of items (9) to (16), wherein the crystalline pravastatin sodium comprises or essentially consists of pravastatin sodium in a form showing characteristic peaks in a X-ray diffractogram at 2 θ of 4.0, 6.3, 10.2, 11.8, 16.3, 17.3, 18.0 and 20.0 +/- 0.2°, preferably +/- 0.1 ° of the 2 θ values ("form LEK").

Within the meaning of the present invention, the term "essentially" denotes that equal to or more than 80%, preferably equal to or more than 90%, more preferably equal to or more than 95% or 97% and most preferably equal to or more than 99% of the crystalline pravastatin sodium is crystalline pravastatin sodium having the above-described characteristic peaks. In a further preferred embodiment, all of the crystalline pravastatin sodium is crystalline pravastatin sodium having the above-described peaks. This crystalline form of pravastatin sodium is preserved in the pharmaceutical composition during processing for obtaining pharmaceutical formulations; it can be preserved also during storage, at least for a substantial time period. In other words, polymorph forms different from the above-mentioned form are only present in trace amounts in the crystalline pravastatin sodium. Trace amounts of polymorph forms different from the above-mentioned form can be measured using X-ray powder diffraction (XRPD). A quantification of such trace amounts can e.g. be carried out by preparing a calibration curve that is based on a mixture of a polymorph which might be present in trace amounts in the pharmaceutical composition and "form LEK".
(18) The pharmaceutical composition according to any of the preceding items, wherein the non-acidic protective coating does not comprise a polymer having an acidic group.
(19) The pharmaceutical composition according to any of the preceding items, wherein the non-acidic protective coating comprises a film forming polymer selected from the group consisting of povidone (polyvinylpyrrolidone), polyethylene glycol, polyvinylpyrrolidone, polyvinylalcohol, hydroxypropylcellulose, methylcellulose, ethyl cellulose, hydroxymethyl cellulose, hydroxypropyl methylcellulose, polyvinyl acetal diethylaminoacetate, and mixtures thereof; and optionally pharmaceutically acceptable excipients selected from the group consisting of plasticizers, pigments, titanium dioxide, anti-adherents, and mixtures thereof. Preferably, the non-acidic protective coating comprises povidone.
(20) The pharmaceutical composition according to any of the preceding items, wherein the non-acidic protective coating comprises polyvinylpyrrolidone, talc, and dibutylsebacate.
(21) The pharmaceutical composition according to any of the preceding items, wherein the enteric coating comprises an enteric polymer selected from the group consisting of cellulose acetate phthalate, hydroxypropyl methylcellulose phthalate, polyvinyl acetate phthalate, carboxymethyl ethylcellulose, co-polymerized methacrylic acid/methacrylic acid methyl esters and mixtures thereof; and optionally pharmaceutically acceptable excipients selected from the group consisting of plasticizers, pigments, titanium dioxide, anti-adherents, and mixtures thereof.
(22) The pharmaceutical composition according to any of the preceding items, wherein the enteric coating comprises propylene glycol, talc, and polymethacrylic acid/methyl methacrylate (Eudragit^{®} L 100-55).
(23) A dosage form comprising the pharmaceutical composition according to any of the preceding items, wherein the dosage form represents a solid oral dosage form, preferably the dosage form is a tablet or a capsule.
(24) The dosage form according to item (23), wherein the dosage form is coated with a coating that is not an enteric coating, and, optionally, further comprises a coating containing pharmaceutically acceptable excipients.
(25) A process for the preparation of pellets containing crystalline pravastatin sodium comprising the steps of:
   a) providing a composition comprising pravastatin sodium, crospovidone and a solvent, further preferred, comprising pravastatin sodium, crospovidone, lactose (such as lactose monohydrate), magnesium oxide, povidone and a solvent,
   b) granulating the composition of step a),
   c) extruding the granulate of step b), and
   d) spheronizing the extrudate of step c).

In a preferred embodiment, the size of the pellets is as described in items (1) or (4).
Preferably, the pellet has an average size in the range of from about 3000 µm to about 400 µm, preferably in the range of from about 2500 µm to about 400 µm, more preferably in the range of from about 2000 µm to about 400 µm, more preferably in the range of from about 1800 µm to about 500 µm, more preferably in the range of from about 1600 µm to about 600 µm, more preferably in the range of from about 1500 µm to about 650 µm, more preferably in the range of from about 1400 µm to about 700 µm, and most preferably in the range of from 1300 µm to about 700 µm.
(26) A process for the preparation of microparticles containing crystalline pravastatin sodium, comprising the steps of:
   a) providing a composition comprising crystalline pravastatin sodium, preferably crystalline pravastatin in the "form LEK", hydroxypropylcellulose, and a solvent, preferably further comprising magnesium oxide and magnesium stearate,
   b) processing the composition of step a) by dissolving the hydroxypropylcellulose in the solvent, and by dispersing the pravastatin sodium and the preferably comprised magnesium stearate and magnesium oxide in the obtained hydroxypropylcellulose solution,
   c) subsequently, the composition is mixed in a non miscible phase to obtain an emulsion, and
   d) evaporating the solvent.

In a preferred embodiment, the size of the microparticles is as described in items (1) or (4).
Preferably, the microparticle has an average size in the range of from less than 400 µm to about 50 µm, preferably in the range of from about 350 µm to about 75 µm, more preferably in the range of from about 350 µm to about 100 µm, more preferably in the range of from about 350 µm to about 120 µm, more preferably in the range of from about 320 µm to about 150 µm, and most preferably in the range of from 300 µm to about 150 µm.
(27) The process according to item (26), wherein the solvent, which is preferably volatile and immiscible with the liquid vehicle phase, is selected from the group consisting of acetone, mixture of acetone and ethanol, mixture of acetone and methanol, methylene chloride, dichlorometane, preferably, the solvent is acetone.
(28) The process according to any of the preceding items (25) to (27), wherein the polymorphic pravastatin sodium has characteristic peaks in an X-ray diffractogram at 2 θ of 4.0, 6.3, 10.2, 11.8, 16.3, 17.3, 18.0 and 20.0 +/- 0.2°, preferably +/- 0.1 °, of the 2 θ values ("form LEK").
(29) Use of a process according any of items (25) to (28) for the preparation of a pharmaceutical composition.
(30) Use of a pharmaceutical dosage form or composition according to any of items (1) to (22) for the prevention or treatment of hypercholesterolemia and hyperlipidemia.

### Detailed description of the invention

The present invention is now described in more detail by preferred embodiments and examples, which are however presented for illustrative purpose only and shall not be understood as limiting the scope of the present invention in any way.

The therapeutic efficacy of any drug depends to a considerable extent on the design of its pharmaceutical formulation. The physico-chemical attributes and bio-pharmacological characteristics account for the formulation of a stable and bioavailable pharmaceutical composition.

Pravastatin sodium is relatively polar and hydrophilic in nature which is susceptible for instance to heat, light and moisture. It is also sensitive to a low pH environment and is very unstable at pH 3 or less as for instance found in the stomach, wherein the hydroxy acids degrade to form primarily lactone and an inactive isomer, 3-a-hydroxy-isopravastatin (Triscari J. et. al.; J. Clin. Pharmacol, 35: 142 (1995)). The instability of pravastatin, in particular its acid sensitivity, can cause significant disadvantages, such as a reduced efficacy and a reduced effectiveness. Following oral administration, pravastatin can be reduced, which results in degradation. Furthermore, due to extensive degradation in acidic environment, such as in the stomach, the bioavailability of pravastatin can be reduced. If kept unprotected, pravastatin undergoes non-enzymatic conversion in the stomach to a relatively inactive metabolite. Consequently, a significant portion of the active ingredient never reaches the site of action in the liver.

Pravastatin exists in different polymorph forms. Polymorphs are forms of the same substance, e.g. of the same active ingredient such as pravastatin, with a difference in crystal packing. This crystal packing can have different level of orders, i.e. a different crystallinity. The higher the level of order, the better is the crystallinity of the respective substance. A lower crystallinity leads to broadened peaks in a X-ray diffractogram, whereas narrow peaks are indicative of a high level of order, i.e a higher crystallinity.

In general, it is desirable to incorporate into a composition a pharmaceutically active ingredient (API) having an improved crystallinity, i.e. having an orderly structure of significant range, which can e.g. be characterized by an X-ray diffraction pattern exhibiting narrow peaks. A better crystallinity may lead to an improved chemical stability of the API, and, therefore, to an improved stability of the pharmaceutical composition comprising this API.

It is known that APIs such as crystalline pravastatin do not maintain their specific crystalline form e.g. during the manufacturing process of the pharmaceutical composition or during storage. Rather, unprotected APIs are conversed into one or more other polymorph forms, whereby a first polymorph form will at least partially convert into at least one other polymorph form e.g. when exposed to adverse environmental influences such as humidity or temperature, and/or by exposure to certain excipients. Such a conversion can be observed and measured by detecting and quantitatively assessing the polymorphs present by any suitable technique that is known to a skilled person, for example a suitable spectroscopic technique such as X-ray powder diffraction (XRPD), Raman or IR, or a suitable thermochemical technique such as differential scanning calorimetry (DSC). The most suitable technique is XRPD, since it allows for the accurate measuring of the relative intensities (area) of peaks specific for each specific polymorph even in mixtures with excipients. It is also possible to use the DSC method, which is a cheap, fast, useful and reliable method for determining of the polymorph form of an API alone, where one can compare the peaks on the thermograms which may be at different temperature for different polymorphs, but is also useful for determination of crystalline form for API in mixtures with other excipients.

It is known that the pravastatin sodium forms D and H as named in WO 01/43723 convert to forms A, H, H1, I, J and K as named in WO 01/43723 by treating with alcohol. It is also stated in WO 01/43723 that any form, except B or D would transform into form D by exposing to 120°C. Form D is characterized by three broad peaks between about 2° and 12° 2 θ and one very broad peak extending from about 15° to 25° 2 8 in its X-ray powder diffraction pattern, i.e. it has a undesirably poor crystalline order. It is also known that the pravastatin sodium "form LEK", characterized by significant peaks having half-value widths below 2° 2 θ in its X-ray powder diffraction pattern, can completely or partially transform into form D, using wet granulation under specific conditions.

It has been unexpectedly found within the present invention that crystalline pravastatin sodium, in particular pravastatin sodium that is present in a specific form showing characteristic peaks in an X-ray diffractogram at 2 θ of 4.0, 6.3, 10.2, 11.8, 16.3, 17.3, 18.0 and 20.0 +/- 0.2° of the 2 θ values (in the following "form LEK"), can be prevented from being conversed to any other polymorph.

Form LEK exhibits an improved, high crystallinity compared to other crystal forms of pravastatin. This improved crystallinity can for instance be deduced from a colourless or pale yellow appearance and from crystals that are in the form of needles or radiating clusters. The crystal shape can be observed under the microscope, for example when observation is carried out under 400-fold magnification. The melting point of the above-mentioned form LEK is between 170°C and 174°C, preferably between 172°C and 174°C. In addition, the signals obtained in an X-ray diffraction measurement (Cu-Kα, 2 θ) have sharp and distinct peaks which are defined by a small half-value width, which also confirms a high degree of crystallinity. Within the meaning of the present invention, the term "half-value width" denotes the value of the 2 θ-range of one peak at the half height or magnitude of the respective peak, preferably the half-value width is 2° or below, and more preferably 1 ° or below. The crystals of pravastatin sodium being the form LEK have a particularly improved crystallinity and, thus, stability at normal conditions and purity compared to other polymorphs of pravastatin, in particular of pravastatin sodium.

Moreover, it is expected that the pharmaceutical composition according to the present invention provides for a decreased biovariability. Without being wished to be bound by any theory it is assumed that the size of the core(s) comprised by a pharmaceutical composition at least partially contributes to a significantly decreased biovariability. With this respect it is expected that with decreasing size of the cores containing the crystalline pravastatin sodium the biovariability is decreased, combined with an increase in bioavailability, leading for instance to an improved effectiveness of the API such as the pravastatin sodium. Additionally, the decreased biovariability is also due to the uniform distribution of the cores according to the present invention after the release of said cores from the dosage form. It is assumed that these cores are uniformly distributed in the stomach, from which they constantly proceed into the small intestine, therefore allowing a more constant absorption of pravastatin. This, in turn, might for instance lead to an improved effectiveness, e.g. due to the constant, uniform distribution of the pravastatin in the body. The biovariability is further decreased by protecting the pravastatin against presystemic degradation in stomach by using an enteric coating. The pellets and microparticles of pravastatin sodium which are coated with such a gastroresistant coating can then e.g. be formulated into the final dosage form, for example capsules or tablets with delayed release of pravastatin in alkaline environment of intestinum.

Furthermore, it has been found that when the particles size is reduced in order to decrease the biovariability, the extent of degradation and conversion of the pravastatin and crystalline form of pravastatin, respectively, could increase. Without wishing to be bound to any theory, it is believed that the particle size reduction leads to a less favorable (increased) ratio of surface/volume of the core containing the API. This means that the contact between the surface of the coating and the API is increased. However, the enteric coating having acidic groups also contributes to the degradation and conversion of the crystalline pravastatin. It has been found that it is beneficial to use a protective coating between the enteric coating and the core in order to protect the API from degradation and conversion caused by contact with the enteric coating. This is also important when reducing the particle size.

It has further unexpectedly been found that when pravastatin is used in crystalline form, particularly in a specific polymorphic form such as the LEK form, the composition of the core strongly influences the stability of the crystalline/polymorphic form of pravastatin. The core compositions as described herein are suitable for stabilizing polymorphic forms of pravastatin, in particular the polymorphic form of pravastatin ("form LEK") having the characteristic peaks at 2 θ of 4.0, 6.3, 10.2, 11.8, 16.3, 17.3, 18.0 and 20.0 +/- 0.2°, preferably +/- 0.1°.

The present invention therefore relates to a pharmaceutical composition comprising one or more cores, an acidic enteric coating and a non-acidic protective coating between the core and the enteric coating, wherein the cores have a specific average particle size, and comprising a specific polymorphic form of pravastatin sodium.

According to the invention, pravastatin is used in crystalline form. In general, pravastatin can be used in one polymorphic form or as a mixture of polymorphic forms. Preferably, the pravastatin which is used according to the present invention comprises the specific polymorphic form of pravastatin sodium showing characteristic peaks in an X-ray diffractogram at 2 θ of 4.0, 6.3, 10.2, 11.8, 16.3, 17.3, 18.0 and 20.0 +/- 0.2° of the 2 θ values. Further preferred, the pravastatin comprises only the specific polymorphic form. Preferably, the pharmaceutical composition according to the present invention contains crystal form D, which is characterized by three broad peaks between about 2° and 12° 2 θ and one very broad peak extending from about 15° to 25° 2 θ in its X-ray powder diffraction pattern, in an amount of less than 1.5%, preferably of less than 1 %, and further preferably of less than 0.7%. In a further preferred embodiment, the presence of crystal form D in the pharmaceutical composition according to the invention can not be detected by a suitable technique, such as XRPD technique. In other words, the amount of crystal form D in the pharmaceutical composition according to the invention is below the level of detection (LOD). Further preferred, the pharmaceutical composition does not contain crystal form D of pravastatin at all, which means that not all three broad peaks between about 2° and 12° 2 θ and the very broad peak extending from about 15° to 25° 2 θ can be identified in an X-ray diffractogram of the core composition, or of the core composition together with the coating composition. Polymorphic forms of e.g. pravastatin being present in the core, which can e.g. be in the form of a microparticle or of a pellet, can be distinguished in X-ray diffractograms of the core using XRPD technique. For this purpose, for instance a so-called placebo core, e.g. a core that does contain the excipients however not the API (e.g. pravastatin sodium in "form LEK") of the core whose impurities are to be measured is prepared. Then, the XRPD pattern of this placebo core is measured. The measured XRPD pattern of the placebo is then count out of the XRPD pattern of the core. What is left is the pravastatin sodium form. By using a calibration curve, the ration of form LEK/form D or other impurities can be determined reliably. It is not necessary to remove the coating that might be present on the core prior to the XRPD analysis. The coating can be included in the placebo preparation.

Excipients that might be present in the core to be analyzed can be determined by any technique that is known to a skilled person. Such techniques include for instance HPLC (high performance liquid chromatography), ICP-MS (Inductively coupled plasma mass spectrometry) for excipients containing metals), Raman spectroscopy, IR (infrared spectroscopy), NIR (near infrared spectroscopy); scanning electron microscopy (SEM), or size exclusion chromatography.

The polymorphic form LEK can be prepared as described in US 7,078,558 e.g. by dissolving of pravastatin sodium in methanol and, while stirring, adding of ethyl acetate and cooling the resuling solution to 8°C. Other polymorphic forms of pravastatin can be prepared as e.g. described in US 7,262,218.

The amount of pravastatin in the cores preferably is in a range of 20 to 60 wt.-% based on the total weight of the core composition. It is also preferred that the amount of pravastatin is between 15 and 55 wt.-% based on the total weight of the core with the non-acidic protective coating and the acidic enteric coating. It is also possible that (a) further API(s) is/are present in the cores.

According to the invention, the term "core" refers to solid composition containing a polymer matrix and the API. Preferably, the core is in the form of a pellet or in the form of a microparticle. As described below, the terms "pellet" and "microparticle" refer to cores which differ in at least one of the following: method of preparation, average size of the cores and excipients comprised by the cores. The pellets and micorparticles can be visually distinguished by their respective size. For instance, when compared, the microparticles exhibit a smaller size compared to the size of the pellets. Furthermore, as to the size of the cores, or to the microparticles or pellets, respectively, reference is made e.g. to items (1) and (4) herein. In case of microparticles, the polymer for the polymer matrix is preferably hydroxypropyl cellulose. Furthermore, it is particularly preferred that the core, in particular the core in the form of a pellet, does not contain any microcrystalline cellulose, and that no water is contained in the pellet and/or is used during the manufacturing process of the pellet. In case of pellets, preferably crospovidone is comprised. Preferably, the cores are prepared by using the solvent evaporation method or extrusion/spheronisation process. Preferably, the cores are prepared by using the processes according to the present invention. When using the solvent evaporation method, mainly cores in form of microparticles are obtained. When using the extrusion/spheronisation process, mainly cores in form of pellets are obtained.

In addition to pravastatin, and crospovidone or hydroxypropylcellulose, the cores preferably comprise further excipients and API(s) suitable for preparing the cores having an average size according to the present invention. Further preferred, the cores additionally comprise the preferred excipients as disclosed herein which contribute to the stabilization of the crystalline form LEK in the pharmaceutical composition.

If the cores are in the form of microparticles, the cores preferably comprise pravastatin sodium, hydroxypropylcellulose, magnesium oxide, and magnesium stearate. Suitable amounts of excipients to be used for the solvent evaporation method can be determined by a person skilled in the art. Preferably, the core comprises pravastatin sodium in an amount of 20-60 wt.-%, hydroxypropylcellulose in an amount of 20-60 wt.-%, magnesium oxide in an amount of 5-30 wt.-%, and magnesium stearate in an amount of 5-30 wt.-%, based on the total weight of the core.

If the cores are in the form of pellets, the cores preferably comprise pravastatin sodium, crospovidone, lactose (e.g. lactose monohydrate), magnesium oxide, and povidone. Suitable amounts of excipients to be used for the extrusion/spheronisation process can be determined by a person skilled in the art. Preferably, the cores comprise pravastatin sodium in an amount of 10-40 wt.-%, crospovidone in an amount of 15-50 wt.-%, lactose (e.g. lactose monohydrate) in an amount of 20-60 wt.-%, magnesium oxide in an amount of 1-20 wt.-%, and povidone in an amount of 5-20 wt.-%, based on the total weight of the core.

In addition to the non-acidic protective coating and the enteric coating, the cores according to the invention can contain further coatings, e.g. coatings containing further APIs. With this respect it is possible that such further coating(s) is present between the core and the non-acidic protective coating and/or between the non-acidic protective coating and the enteric coating and/or on the enteric coating. Preferably, however, the cores according to the invention consist of the cores, the non-acidic protective coating and the enteric coating. This means that the cores consist of a homogenous composition containing the API(s) and the excipients, wherein the cores are coated with the non-acidic protective coating and the enteric coating. Further preferred, the thus coated cores are then manufactured into pharmaceutical compositions for instance by compressing into tablets, or filling into capsules.

The coated cores can also be directly used as a medicament. For example the coated cores can be directly administered as a suspension in water to a patient.

The size of the microparticle cores either with or without coating (non-acidic protective coating and optionally acidic enteric coating) can e.g. be determined by using Malvern measurement by determining the d0.1, d0.5 and d0.9 value. A suitable apparatus is for instance the "Mastersizer", Malver UK. In order to determine the size of the microparticle cores, e.g. the measuring cell of this apparatus is filled with ethylacetate. Then, three droplets of Polysorbate 80 and the sample are added. The mixture is mixed at 2000 rpm, and then the size was determined. As mentioned above, it is not necessary to remove coating(s) that might be present on the core(s) prior to the measuring of the size.

The cores, preferably in form of pellets or microparticles, have an average size of equal to or less than about 3000 µm, preferably an average size in the range of from about 3000 µm to about 50 µm, more preferably of from about 2000 µm to about 70 µm, and even more preferably from about 1500 µm to about 100 µm. Further preferred, the cores have an average size in the range of from about 3000 µm to about 400 µm, preferably in the range of from about 2500 µm to about 400 µm, more preferably in the range of from about 2000 µm to about 400 µm, more preferably in the range of from about 1800 µm to about 500 µm, more preferably in the range of from about 1600 µm to about 600 µm, more preferably in the range of from about 1500 µm to about 650 µm, more preferably in the range of from about 1400 µm to about 700 µm, and most preferably in the range of from 1300 µm to about 700 µm.

If the cores are in the form of pellets, the pellets preferably have an average size in the range of from about 3000 µm to about 400 µm, preferably in the range of from about 2500 µm to about 400 µm, more preferably in the range of from about 2000 µm to about 400 µm, more preferably in the range of from about 1800 µm to about 500 µm, more preferably in the range of from about 1600 µm to about 600 µm, more preferably in the range of from about 1500 µm to about 650 µm, more preferably in the range of from about 1400 µm to about 700 µm, and most preferably in the range of from 1300 µm to about 700 µm.

If the cores are in the form of microparticles, the microparticles have an average size in the range of from less than 400 µm to about 50 µm, preferably in the range of from about 350 µm to about 75 µm, more preferably in the range of from about 350 µm to about 100 µm, more preferably in the range of from about 350 µm to about 120 µm, more preferably in the range of from about 320 µm to about 150 µm, and most preferably in the range of from 300 µm to about 150 µm.

The pharmaceutical dosage form according to the invention provides further advantages, e.g. with respect to the dissolution profile and biovariability, if the cores are not present in form of agglomerates prior to the coating of the cores with the non-acidic protective coating. The processes according to the present invention for preparing cores do essentially not provide agglomerates of cores but provide singe pellets or single microparticles. This means that out of 100 produced microparticles or pellets, at most 10, preferably at most 7, more preferably at most 5, and even more preferably at most 3 or at most 1 of the produced cores, i.e. microparticles or pellets, are agglomerated. Most preferred, there are no agglomerated cores, i.e. microparticles or pellets, present. Within the meaning of the present invention, the term "agglomerate" denotes that 2 or more pellets are associated with each other. Whether the pellets are associated or not, i.e. form agglomerates or not, can be determined by any suitable method. For instance, the microparticles or pellets can be inspected visually by using a microscope, and their size can be determined.

According to the invention, the term "non-acidic protective coating" refers to a coating which is applied onto the cores and prevents the cores to be in direct contact with the acidic enteric coating which e.g. contains free carboxyl groups, which otherwise causes degradation of pravastatin during the coating process or during the storage. Preferably, the protective coating does not comprise a polymer having an acidic group. Further preferred, the protective coating does not contain microcrystalline cellulose.

Additionally preferred, the non-acidic protective coating comprises a film forming polymer selected from the group consisting of polyethylene glycol, polyvinylpyrrolidone, polyvinylalcohol, hydroxypropylcellulose, methylcellulose, ethyl cellulose, hydroxymethyl cellulose, hydroxypropyl methylcellulose, polyvinyl acetal diethylaminoacetate, and mixtures thereof; further preferred, the non-acidic protective coating comprises povidone, further preferred it comprises or consists of povidone (polyvinylpyrrolidone), talc and dibutylsebacate.

Additionally preferred, the thickness of the non-acidic protective coating is in a range of from 10 to 100 µm, further preferred in a range of from 15 to 50 µm, even further preferred in a range of from 15 to 30 µm. The thickness of the coating can be determined by using SEM (scanning electron microscopy).

Suitable non-acidic protective coating and methods for applying a non-acidic protective coating onto the cores are well known in the art. In a preferred embodiment, the cores may be coated with non-acidic protective coating as well as with acidic enteric coating by fluid-bed coating, pan coating or other standard techniques known to a person skilled in the art. In a further preferred embodiment, the cores are coated using a fluid bed coater with wurster insert.

According to the invention, the term "acidic enteric coating" refers to a coating which does not dissolve in the stomach and comprises a polymer comprising acidic functional groups. By definition, an enteric polymer or mixture of enteric polymers does not dissolve at a pH below 5. The acidic enteric coating preferably contains an enteric polymer selected from the group consisting of cellulose acetate phthalate, hydroxypropyl methylcellulose phthalate, polyvinyl acetate phthalate, carboxymethyl ethylcellulose, co-polymerized methacrylic acid/methacrylic acid methyl esters and mixtures thereof; and optionally pharmaceutically acceptable excipients selected from the group consisting of plasticizers, pigments, titanium dioxide, anti-adherents, and mixtures thereof. Anionic polymers or copolymers with methacrylic acid as a functional group are sold unter the tradename EUDRAGIT^{®} (Degussa, Germany): EUDRAGIT^{®} L100-55 (sold in powder form; dissolution above pH 5.5), EUDRAGIT^{®} L100 (sold in powder form; dissolution above pH 6.0) or EUDRAGIT^{®} L12,5 (sold as organic solution, 12.5%; dissolution above pH 6.0), EUDRAGIT^{®} S100 (sold in powder form; dissolution above pH 7.0), or EUDRAGIT^{®} S12,5 (sold as organic solution, 12.5%; dissolution above pH 7.0). Preferably, the enteric polymer which contained in the acidic enteric coating is selected from the group consisting of methacrylic acid/ethylacrylate, methacrylic acid/methyl methacrylate, and methacrylic acid/methylacrylate/methyl methacrylate. Further preferred, polymers that are to be used as aqueous dispersions are not used in order to avoid the use of water in the coating process.

It is particularly preferred that the enteric coating comprises an enteric polymer selected from the group consisting of cellulose acetate phthalate, hydroxypropyl methylcellulose phthalate, polyvinyl acetate phthalate, carboxymethyl ethylcellulose, co-polymerized methacrylic acid/methacrylic acid methyl esters and mixtures thereof; and optionally pharmaceutically acceptable excipients selected from the group consisting of plasticizers, pigments, titanium dioxide, anti-adherents, and mixtures thereof. In general, the amount of enteric polymer applied onto the core or on the protective coating covering the core, respectively, should be such that it provides gastroresistancy. The gastroresistancy of a coating can be tested e.g. by using common dissolution method (for example the US paddle method).

Suitable enteric coating compositions and methods for applying an enteric coating to the cores which are coated with the non-acidic protective coating are well known in the art.

Preferably, the enteric coating comprises propylene glycol, talc, and polymethacrylic acid/methyl methacrylate (Eudragit^{®} L 100-55). Preferably, the enteric coating comprises the enteric polymer in amounts between 30 and 80 wt.-%.

Additionally preferred, the thickness of the acidic enteric coating is in a range of from 10 to 100 µm, further preferred in a range of from 15 to 50 µm, even further preferred in a range of from 20 to 40 µm. The thickness of the coating can be determined by using SEM (scanning electron microscopy).

Considering the increased size of coated pellets and microparticles because of the applied non-acidic protective coating and the acidic enteric coating, the average particle size of the cores comprising the aforementioned coatings, preferably cores in form of pellets or microparticles, have an average size of equal to or less than about 3100 µm, preferably an average size in the range of from about 3100 µm to about 70 µm, more preferably of from about 2000 µm to about 90 µm, and even more preferably from about 1600 µm to about 100 µm. Further preferred, the cores have an average size in the range of from about 3000 µm to about 400 µm, preferably in the range of from about 2500 µm to about 400 µm, more preferably in the range of from about 2000 µm to about 400 µm, more preferably in the range of from about 1800 µm to about 500 µm, more preferably in the range of from about 1600 µm to about 600 µm, more preferably in the range of from about 1500 µm to about 650 µm, more preferably in the range of from about 1400 µm to about 700 µm, and most preferably in the range of from 1300 µm to about 700 µm.

If the cores are in the form of pellets, the pellets comprising the non-acidic protective coating and the acidic enteric coating preferably have an average size in the range of from about 3100 µm to about 400 µm, preferably in the range of from about 2600 µm to about 400 µm, more preferably in the range of from about 2100 µm to about 400 µm, more preferably in the range of from about 1900 µm to about 500 µm, more preferably in the range of from about 1700 µm to about 600 µm, more preferably in the range of from about 1600 µm to about 650 µm, more preferably in the range of from about 1400 µm to about 700 µm, and most preferably in the range of from 1300 µm to about 700 µm.

If the cores are in the form of microparticles, the microparticles have an average size in the range of from less than 500 µm to about 50 µm, preferably in the range of from about 400 µm to about 75 µm, more preferably in the range of from about 350 µm to about 100 µm, more preferably in the range of from about 350 µm to about 130 µm, more preferably in the range of from about 320 µm to about 160 µm, and most preferably in the range of from 300 µm to about 160 µm.

Additionally preferred, the non-acidic protective coating and/or the acidic enteric coating comprises further pharmaceutically acceptable excipients selected from the group consisting of plasticizers, pigments, titanium dioxide, anti-adherents, and mixtures thereof.

As described above, the core composition according to the present invention allows preserving the polymorphic form of pravastatin. Thus, the present invention refers to a pharmaceutical composition comprising one or more cores, an acidic enteric coating and a non-acidic protective coating between the core and the enteric coating, wherein the cores comprise crystalline pravastatin sodium, and crospovidone, preferably crystalline pravastatin sodium, crospovidone, lactose, magnesium oxide, and povidone, wherein the cores are coated with the non-acidic protective coating, and wherein the cores with said non-acidic protective coating are coated with the enteric coating. In this embodiment according to the invention, the cores are preferably in the form of pellets, preferably pellets which have been manufactured by the process as described below. Preferred embodiments of the pellets, the cores, and the coatings are described above. It is expected that the pharmaceutical composition according to the present invention provides for e.g. an improved bioavailability, an increased biovariability and, thus, an improved effectiveness. Without wishing to be bound by any theory it is assumed that this is due to the interaction of said excipients, the crystalline form of pravastatin (in particular if specifically the form LEK) and the form of the core. In particular, it is expected that the above-mentioned excipients, in combination with the specific form LEK and the core in form of a pellet, provides for the best result in terms of stability, biovariability and bioavailability.

Furthermore, the present invention relates to a pharmaceutical composition comprising one or more cores, an acidic enteric coating and a non-acidic protective coating between the core and the enteric coating, wherein the cores comprise pravastatin sodium and hydroxypropylcellulose, preferably, the cores comprise pravastatin sodium, hydroxypropylcellulose, magnesium oxide, and magnesium stearate, wherein the core is coated with the protective coating, and wherein the core with said protective coating is coated with the enteric coating.

In this embodiment according to the invention, the cores are preferably in the form of microparticles, preferably microparticles which have been manufactured by the process as described below. Preferred embodiments of the microparticles, the cores, and the coatings are described above. It is expected that the combination of the specific excipients as mentioned above, namely hydroxypropylcellulose, magnesium oxide, and magnesium stearate, in combination with the crystalline form of pravastatin sodium (in particular if the specific crystalline form LEK) and the core in form of a microparticle, provides for the best result in terms of stability, biovariability and bioavailability.

The present invention also refers to a dosage form comprising the pharmaceutical composition according to any of the preceding claims, wherein the dosage form represents a solid oral dosage form, preferably the dosage form is a tablet or a capsule.

In a preferred embodiment, the dosage form is coated with a coating that is not an enteric coating, and, optionally, the dosage form further comprises a coating containing pharmaceutically acceptable excipients.

According to the invention, the cores in form of pellets are prepared by using an extrusion/spheronisation process. The pellets are prepared using common extrusion and spheronization techniques. Preferred average pellet sizes that can be obtained by using the process according to the invention are described above.

In general, in order to prepare pellets having desired properties by using extrusion-spheronization process, the wetted mass must fulfill certain conditions: during extrusion, the wetted powder material must be able to form a cohesive yet plastic mass that remains homogeneous throughout the extrusion process. For spheronization, a precise balance between plasticity and brittleness of the extrudates is needed to complete the process effectively. Microcrystalline cellulose as main excipient and water as granulating liquid are most widely used for preparation of pellets with extrusion/ spheronization.

However, it has unexpectedly been found that during granulation of crystalline pravastatin sodium and microcrystalline cellulose with water, the polymorphic form of pravastatin is changed even if only small amounts of water were used (i.e. 10 % of water), since pravastatin sodium has a good solubility in water. Therefore, various spheronization aids, alternative excipients and granulation liquid were tested like e.g. L-hydroxypropylcellulose (L-HPC), k-carrageenan, polyplasdone XL, mannitol, lactose, maize starch, Gelucire 50/13, povidone in combination with absolute ethanol and isopropyl alcohol.

It was unexpectedly found that polymorphs of pravastatin, preferably the polymorph "form LEK" can be stabilized when using crospovidone as a core ingredient. A further stabilization can be achieved when using a combination of pravastatin sodium, polyplasdone XL-1 0 (crospovidone), lactose (such as lactose monohydrate), magnesium oxide, povidone and isopropanol as the granulation liquid for the preparation of pellets in order to essentially retain the used initial polymorphic structure of the active ingredient pravastatin sodium, in particular "form LEK". Within the meaning of the present invention, the term "essentially" denotes that equal to or more than 80%, preferably equal to or more than 90%, more preferably equal to or more than 95% or 97% and most preferably equal to or more than 99% of the crystalline pravastatin sodium being present in the pellets has the polymorphic structure of the used initial pravastatin sodium. The initial polymorphic structure is essentially preserved during the process according to the present invention. In other combinations of excipients, recrystallization of pravastatin sodium in form D or other polymorphic forms during preparation was observed to a greater extent, as demonstrated with X-ray diffraction spectroscopy.

Thus, the present invention also relates to a process for the preparation of pellets containing crystalline pravastatin sodium comprising the steps of:
a) providing a composition comprising pravastatin sodium, crospovidone and a solvent, further preferred comprising pravastatin sodium, crospovidone, lactose, magnesium oxide, povidone, and solvent,
b) granulating the composition of step a),
c) extruding the granulate of step b), and
d) spheronizing the extrudate of step c).

Suitable solvents which can be used in the extrusion/spheronization process are known in the art. Preferably, the solvent is selected from the group consisting of absolute (abs.) ethanol, isopropyl alcohol or a mixture of acetone and abs. ethanol, preferably, the solvent is isopropyl alcohol.

It is additionally preferred that the ratio of solvent(s)/dry excipients is between 0.05/1 to 1/1, further preferred between 0.05/1 to 0,9/1 and even further preferred between 0,1/1 to 0,8/1 and most preferably, between 0,1/1 to 0,7/1.

Additionally preferred, the pravastatin sodium is used in the polymorphic form LEK as described above.

In order to obtain cores containing pravastatin sodium having a smaller size, various technological procedures were tested and the influence of the process of preparation on the recrystallization of pravastatin sodium was evaluated. It has unexpectedly been found that the cores in form of microparticles can advantageously be prepared by using the solvent evaporation method. Preferred average sizes of cores in form of microparticles are described above.

The solvent evaporation method is a more complex technological procedure in comparison to the extrusion/spheronization process and is e.g. described in the "Handbook of Pharmaceutical Controlled Release Technology, (Marcel Dekker Inc.), Chapter 16: Microsphere Preparation by solvent evaporation method", or in the "Encyclopedia of pharmaceiutical technology (Informa Healthcare), Chapter Microsphere Technology and Applications". A person skilled in the art is able to carry out such a method.

The advantage of this method is that a smaller size of particles can be provided compared to the size of the particles that are prepared by using the extrusion/spheronization process. The particles comprise the API which is present in the matrix polymer or core polymer, respectively. Briefly, the matrix polymer is dissolved in an organic solvent, and the other components are then dispersed or dissolved in the polymer solution. This dispersion is dispersed in nonmiscible phase (e.g. liquid paraffin) to form emulsion droplets. Continuous mixing at elevated temperature, e.g. at a temperature of 35 - 50°C, most preferably 40°C, is employed to evaporate the more volatile organic solvent and leave the solid polymer- drug particles suspended in the suspension which is formed. The particles are finally filtered off from the suspension.

Furthermore, it was found that a very good stabilization of the polymorph of pravastatin can be achieved when using hydroxypropycellulose as a polymer in the solvent evaporation method. A further improvement can be achieved when using the preferred combination of components comprising hydroxypropycellulose, magnesium oxide as alkalizer, magnesium stearate as surface active agent and a solvent. Thereby, as described above, the used initial polymorphic structure of the active ingredient pravastatin sodium can be retained, at least essentially, and in particular "form LEK". Within the meaning of the present invention, the term "essentially" denotes that equal to or more than 80%, preferably equal to or more than 90%, more preferably equal to or more than 95% or 97% and most preferably equal to or more than 99% of the crystalline pravastatin sodium being present in the microparticles has the polymorphic structure of the used initial pravastatin sodium.

Cosequently, the process according to the invention for the preparation of microparticles containing crystalline pravastatin sodium comprises the steps of:
a) providing a composition comprising crystalline pravastatin sodium, hydroxypropylcellulose, and a solvent and, preferably further comprising magnesium oxide, and magnesium stearate,
b) processing the composition of step a) by dissolving the hydroxypropylcellulose in the solvent, and by dispersing the pravastatin sodium and the preferably comprised magnesium stearate and magnesium oxide in the obtained hydroxypropylcellulose solution,
c) subsequently, the composition is mixed in a non miscible phase to obtain an emulsion, and
d) evaporating the solvent.

Suitable solvents which can be used in the solvent evaporation method are known in the art. Preferably, the solvent is selected from the group consisting of acetone, mixture of acetone and ethanol, mixture of acetone and methanol, methylene chloride, and dichlorometane. Preferably, the solvent is acetone.

It is additionally preferred that the ratio of solvent(s)/dry excipients is between 20/1 and 2/1, preferably between 10/1 and 5 /1.

Preferred amounts of excipients and pravastatin, and other preferred parameters are described above with respect to the pharmaceutical composition. It is particularly preferred that pravastatin sodium is used in the polymorphic form LEK as described above.

Microparticles of pravastatin sodium prepared with solvent evaporation have a smaller particle size than other cores which contain pravastatin but which have been prepared by using a different method of preparation. The microparticles according to the invention are expected to be better distributed in the gastrointestinal tract and are expected to have an improved bioavailability, a decreased biovariability and an improved stability as to the crystalline pravastatin sodium (form LEK).

According to the invention, the cores, preferably in form of pellets or microparticles, are coated with a protective coating such as a non-acidic protective coating, and, subsequently with an acidic enteric coating.

The cores can be coated by using any known methods. Preferably, the cores are coated in a fluidized bed (Wurster process), firstly e.g. with intermediate protective povidone coating and secondly with a gastroresistant enteric coating of polymethacrylic acid - methyl methacrylate (Eudragit® L100-55).

The resulting cores with the non-acidic protective coating and the enteric coating can then e.g. be compressed into tablets or filled in capsules.

The present invention also relates to the use of a process according to the invention for the preparation of a pharmaceutical composition.

The present invention also refers to the use of a pharmaceutical dosage form or pharmaceutical composition according to the invention for the prevention or treatment of hypercholesterolemia and hyperlipidemia.

### EXAMPLE 1 (comparative example): Wet granulation of Pravastatin sodium and microcrystalline cellulose with water

A dry mixture of pravastatin sodium (form LEK) and microcrystalline cellulose in a ratio of 1:2 was wetted with 10 % (Example 1 a), 20 % (Example 1 b) and 40 % (Example 1 c) of water and granulated. The resulting granulate was wetted for 5 hours and then dried for 12 h at 40°C. The polymorphic structure of pravastatin sodium was characterized by using X-ray powder diffraction (XPRD) analysis. Samples were measured on apparatus X'Pert PRO MPD (from Panalytical) by reflex techniques at two conditions:
a. CuKα radiation, range from 2° to 40° 2 θ, step 0,033° 2 θ
b. CuKα radiation, range from 2° to 12° 2 θ, step 0,05° 2 θ, 2000 s/step

In all samples pravastatin sodium (form LEK) recrystallized into other polymorphic forms.

### EXAMPLE 2: Wet granulation of Pravastatin sodium and different excipients with absolute ethanol and isopropyl alcohol

| Sample | Composition of mixture | Granulation liquid | Ratio of dry mixture : gran. liquid |
|---|---|---|---|
| Example 2_1 | Pravastatin sodium : microcrystalline cellulose = 1:0,5 | Isopropyl alcohol | 1:1 |
| Example 2_2 | Pravastatin sodium : lactose monohydrate = 1:2 | Isopropyl alcohol | 1:1 |
| Example 2_3 | Pravastatin sodium : lactose anhydrous = 1:2 | Isopropyl alcohol | 1:1 |
| Example 2_4 | Pravastatin sodium | Isopropyl alcohol | 1:1,5 |
| Example 2_5 | Pravastatin sodium : k-carrageenan = 1:3 | Absolut (Abs.). ethanol | 2:1 |
| Example 2_6 | Pravastatin sodium : maize starch = 1:3 | Abs. ethanol | 2:1 |
| Example 2_7 | Pravastatin sodium : mannitol = 1:3 | Abs. ethanol | 2:1 |
| Example 2_8 | Pravastatin sodium : polyplasdone XL = 1:3 | Abs. ethanol | 2:1 |
| Example 2_9 | Pravastatin sodium : microcrystalline cellulose = 1:3 | Abs. ethanol | 2:1 |
| Example 2_10 | Pravastatin sodium : k-carrageenan = 1:3 | Isopropyl alcohol | 2:1 |
| Example 2_11 | Pravastatin sodium : maize starch = 1:3 | Isopropyl alcohol | 2:1 |
| Example 2_12 | Pravastatin sodium : mannitol = 1:3 | Isopropyl alcohol | 2:1 |
| Example 2_13 | Pravastatin sodium : polyplasdone XL = 1:3 | Isopropyl alcohol | 2:1 |
| Example 2_14 | Pravastatin sodium : microcrystalline cellulose = 1:3 | Isopropyl alcohol | 2:1 |
| Example 2_15 | Pravastatin sodium : Gelucire 50/13 = 1:3 | / | / |

Example 2_1 - 2_14: A dry mixture of pravastatin sodium (form LEK) and selected excipients were wetted with a granulation liquid and granulated. The granulate was wetted for 52 hours and then dried overnight at 45°C.

Example 2_15: Pravastatin sodium (form LEK) was mixed with melted Gelucire 50/13 (Gelucire excipients are polyethylene glycol glycerides composed of mono-, di- and triglycerides and mono- and diesters of polyethylene glycol (PEG) and allowed to solidify.

The polymorphic structure of pravastatin sodium was characterized with X-ray powder diffraction (XPRD) analysis. Samples were measured on apparatus X'Pert PRO MPD by reflex techniques at two conditions:
a. CuKα radiation, range from 2° to 40° 2θ, step 0,033° 2θ
b. CuKα radiation, range from 3° to 12° 2θ, step 0,05° 2θ, 2000 s/step

| Sample | Result of XPRD analysis |
|---|---|
| Example 2_1 | Form LEK |
| Example 2_2 | Form LEK + trace of other forms |
| Example 2_3 | Form LEK + trace of other forms |
| Example 2_4 | Form LEK |
| Example 2_5 | Amorphous + trace of other forms |
| Example 2_6 | Form LEK + trace of other forms |
| Example 2_7 | Form D + Form LEK |
| Example 2_8 | Form LEK |
| Example 2_9 | Form LEK + Form D |
| Example 2_10 | Amorphous |
| Example 2_11 | Form D + Form LEK + trace of other forms |
| Example 2_12 | Form LEK + Form D |
| Example 2_13 | Form LEK |
| Example 2_14 | Form LEK + Form D |
| Example 2_15 | Form LEK |

### EXAMPLE 3: Preparation of Pravastatin sodium pellets using the extrusion spheronization process

| | Ex. 3a | Ex. 3b | Ex. 3c | Ex. 3d | Ex. 3e |
|---|---|---|---|---|---|
| | % | % | % | % | % |
| Pravastatin Sodium | 20.0 | 20.0 | 20.0 | 20.0 | 20.0 |
| Microcrystalline cellulose | 10.0 | 10.0 | - | - | 10.0 |
| Lactose monohydrate | 36.4 | 36.4 | 46.4 | 39.4 | 36.4 |
| Magnesium oxide | 5.0 | 5.0 | 5.0 | 5.0 | 5.0 |
| L-hydroxypropylcellulose | 18.0 | 18.0 | 18.0 | 25.0 | 18.0 |
| Crosscarmellose sodium | 5.0 | 5.0 | 5.0 | 5.0 | 5.0 |
| Povidone | 5.0 | 5.0 | 5.0 | 5.0 | 5.0 |
| Polisorbate 80 | 0.6 | 0.6 | 0.6 | 0.6 | - |
| Absolute ethanol | 25.0 | 21.5 | 25.0 | 25.0 | - |
| Acetone : Absolute ethanol (1:1) | - | - | - | - | 26.67 |

| | Ex. 3f | Ex. 3g | Ex. 3h | Ex.3i | Ex. 3j |
|---|---|---|---|---|---|
| | % | % | % | % | % |
| Pravastatin Sodium | 20.0 | 20.0 | 20.0 | 20.0 | 20.0 |
| Polyplasdone XL-10 | 25.0 | 25.0 | 25.0 | 25.0 | 25.0 |
| Lactose monohydrate | 35.0 | 40.0 | 37.5 | 40.0 | 40.0 |
| Magnesium oxide | 5.0 | 5.0 | 5.0 | 5.0 | 5.0 |
| Crosscarmellose sodium | 5.0 | 5.0 | 5.0 | - | - |
| Povidone | 10.0 | 5.0 | 7.5 | 10.0 | 10.0 |
| Absolute ethanol | 26.67 | - | - | 43,3 | - |
| Acetone : Absolute ethanol (1:1) | - | 40.0 | - | - | - |
| Isopropyl alcohol | - | - | 23.33 | - | 43.3 |

### Preparation of pellets:

Pravastatin sodium was sieved through a 0.50 mm sieve and mixed with polyplasdone XL-10, lactose, magnesium oxide and povidone were mixed in planetary mixer (2 min, 25 o/min). The mixture was granulated with isopropyl alcohol (25 o/min) and additionally mixed (1 min, 300/min).

Granulate was extrudated through mesh 0.70 mm. Extrudates were then spheronized under different conditions to produce as much spheronized pellets as possible.

Polymorphic structure of pravastatin sodium was characterized with X-ray powder diffraction (XPRD) analysis.

| Sample | Result of XPRD analysis |
|---|---|
| Example 3a | Form D + Form LEK |
| Example 3b | Form D + Form LEK |
| Example 3c | Form D + Form LEK |
| Example 3d | Form D + Form LEK |
| Example 3e | Form D + Form LEK |
| Example 3f | Form LEK |
| Example 3g | Form LEK |
| Example 3h | Form LEK + trace of other forms |
| Example 3i | Form LEK + trace of other forms |
| Example 3j | Form LEK |

### EXAMPLE 4: Preparation of Pravastatin sodium microparticles with solvent evaporation method

| | Example 4 |
|---|---|
| | % |
| Pravastatin Sodium | 40.0 |
| Hydroxypropylcellulose | 40.0 |
| Magnesium oxide | 10.0 |
| Magnesium stearate | 10.0 |

Hydroxypropycellulose is dissolved in acetone. This solution is cooled to 5-10°C. Magnesium stearate is added and the dispersion is mixed with a magnetic stirrer for 5 min. Then, magnesium oxide is added and the dispersion is homogenized with a rotor stator homogenizer. Pravastatin sodium, sieved through 100 µm, is added and additionally homogenized with a rotor stator homogenizer.

The prepared dispersion is mixed in cooled liquid paraffin (5-10°C) to obtain an emulsion. It is mixed for 5 min at 5-10°C. Then, the emulsion is slowly heated during constant mixing up to 40°C. The emulsion is vigorously mixed at 40°C for 1 hour to evaporate the acetone. Consequently, matrix microparticles of hydroxypropycellulose, pravastatin sodium, magnesium stearate and magnesium oxide are formed. Afterwards, the dispersion is cooled down to 25°C. The microparticles were isolated from liquid paraffin, washed with methylcyclohexane and dried overnight in a vacuum dryer.

The polymorphic structure of pravastatin sodium was characterized with X-ray powder diffraction (XPRD) analysis. Initial polymorphous structure (form Lek) is preserved during preparation of the microparticles.

### EXAMPLE 5: Coating of pravastatin sodium pellets with gastroresistant (enteric) coating

| | Example 5a | Example 5b | Example 5c |
|---|---|---|---|
| Cores | | | |
| Example 3j (pellets) | 200.00 mg | - | - |
| Example 4 (microparticles) | - | 100.00 mg | 100.00 mg |

| Subcoat | | | |
|---|---|---|---|
| Povidone | 14.40 mg | 9.00 mg | 9.00 mg |
| Talc | 24.80 mg | 15.50 mg | 15.50 mg |
| Dibutylsebacate | 0.80 mg | 0.50 mg | 0.50 mg |

| Coat | | | |
|---|---|---|---|
| Eudragit L100-55 | 35.99 mg | 37.50 mg | 112.50 mg |
| Propylene Glycol | 7.22 mg | 7.53 mg | 22.59 mg |
| Talc | 17.99 mg | 18.75 mg | 56.25 mg |

**Preparation of subcoating dispersion:** Povidone was dissolved in isopropanol. Dibutylsebacate was added and dispersion was additionally mixed, finally talc was added to the dispersion and mixed.

**Preparation of coating dispersion:** Eudragit^{®} L100-55 was dissolved in part of isopropanol. Propylene glycol was added and the dispersion was mixed. Talc was dispersed in the isopropanol and this dispersion is added to Eudragit^{®} L100-55 solution. Cores were coated using fluid bed coater with wurster insert. The gastroresistant particles of pravastatin sodium are filled in capsules or compressed in tablets.

## Claims

1. A pharmaceutical composition comprising one or more cores, an acidic enteric coating and a non-acidic protective coating between the core(s) and the enteric coating, wherein the core(s) has (have) an average size of equal to or less than about 3000 µm, and wherein the core(s) comprise(s) pravastatin sodium in a form showing characteristic peaks in an X-ray diffractogram at 2 θ of 4.0, 6.3, 10.2, 11.8, 16.3, 17.3, 18.0 and 20.0 +/- 0.2° of the 2 θ values.

2. The pharmaceutical composition according to claim 1, wherein said core is in the form of a pellet or of a microparticle.

3. The pharmaceutical composition according to claim 1 or 2, wherein if said core is in the form of a pellet the pellet has an average size in the range of from about 3000 µm to about 400 µm,
and wherein if said core is in the form of a microparticle the microparticle has an average size in the range of from less than 400 µm to about 50 µm.

4. The pharmaceutical composition according to any of the preceding claims, wherein the core comprises pravastatin sodium, and hydroxypropylcellulose or crospovidone.

5. A pharmaceutical composition comprising one or more cores, an acidic enteric coating and a non-acidic protective coating between the core and the enteric coating, wherein the core comprises crystalline pravastatin sodium, crospovidone, lactose, magnesium oxide, and povidone,
wherein the core is coated with the non-acidic protective coating, and
wherein the core with said non-acidic protective coating is coated with the enteric coating.

6. A pharmaceutical composition comprising one or more cores, an acidic enteric coating and a non-acidic protective coating between the core and the enteric coating, wherein the core comprises crystalline pravastatin sodium, hydroxypropylcellulose, magnesium oxide, and magnesium stearate,
wherein the core is coated with the non-acidic protective coating, and
wherein the core with said non-acidic protective coating is coated with the enteric coating.

7. The pharmaceutical composition according to claim 6, wherein the core comprises pravastatin sodium in an amount of 20-60 wt.-%, hydroxypropylcellulose in an amount of 20-60 wt.-%, magnesium oxide in an amount of 5-30 wt.-%, and magnesium stearate in an amount of 5-30 wt.-%, based on the total weight of the core.

8. The pharmaceutical composition according to any of claims 5 to 7, wherein the crystalline pravastatin sodium comprises or essentially consists of pravastatin sodium in a form showing characteristic peaks in a X-ray diffractogram at 2 θ of 4.0, 6.3, 10.2, 11.8, 16.3, 17.3, 18.0 and 20.0 +/- 0.2° of the 2 θ values.

9. The pharmaceutical composition according to any of the preceding claims, wherein the enteric coating comprises an enteric polymer selected from the group consisting of cellulose acetate phthalate, hydroxypropyl methylcellulose phthalate, polyvinyl acetate phthalate, carboxymethyl ethylcellulose, co-polymerized methacrylic acid/methacrylic acid methyl esters and mixtures thereof; and optionally pharmaceutically acceptable excipients selected from the group consisting of plasticizers, pigments, titanium dioxide, anti-adherents, and mixtures thereof.

10. A dosage form comprising the pharmaceutical composition according to any of the preceding claims, wherein the dosage form represents a solid oral dosage form.

11. A process for the preparation of pellets containing crystalline pravastatin sodium comprising the steps of:
a) providing a composition comprising pravastatin sodium, crospovidone, and a solvent,
b) granulating the composition of step a),
c) extruding the granulate of step b), and
d) spheronizing the extrudate of step c).

12. A process for the preparation of microparticles containing crystalline pravastatin sodium, comprising the steps of:
a) providing a composition comprising crystalline pravastatin sodium, hydroxypropylcellulose, and a solvent,
b) processing the composition of step (a) by dissolving the hydroxypropylcellulose in the solvent, and by dispersing the pravastatin sodium in the obtained hydroxypropylcellulose solution,
c) subsequently, the composition is mixed in a non miscible phase to obtain an emulsion, and
d) evaporating the solvent.

13. Use of a process according to claim 11 or 12 for the preparation of a pharmaceutical composition.

14. Use of a pharmaceutical dosage form or pharmaceutical composition according to any of claims 1 to 10 for the prevention or treatment of hypercholesterolemia and hyperlipidemia.
